# EUROPEAN PATENT APPLICATION

(11) **EP 4 691 351 A1**
(43) Date of publication of application: **11.02.2026**
(21) Application number: 24780675.5
(22) Date of filing: 28.03.2024
(51) Int. Cl.: A61B 5/02

(54) **PULSE WAVE SENSOR**

(30) Priority: 30.03.2023 US 202363493109 P; 31.07.2023 JP 2023124909
(71) Applicant: Minebea Mitsumi Inc., Kitasaku-gun, Nagano 3890293 (JP)
(72) Inventor: HARADA, Koshi, Kitasaku-gun, Nagano 389-0293 (JP); OKA, Hiroyuki, Kitasaku-gun, Nagano 389-0293 (JP); TOKUDA, Yuta, Kitasaku-gun, Nagano 389-0293 (JP)
(74) Representative: Winter, Brandl - Partnerschaft mbB
(86) International application number: PCT/JP2024/012720
(87) International publication number: WO 2024/204575

(57) **Abstract**

A pulse wave sensor is a pulse wave sensor to be used in contact with a body of a measurement target. The pulse wave sensor includes a flexure element having a first surface that is on a measurement target side, and a second surface located opposite to the first surface; at least one detection element disposed on the second surface, and configured to detect strain of the flexure element and/or pressure applied to the flexure element; and a cover member mounted or formed on the first surface. The cover member includes a recess opening toward the first surface of the flexure element, and a protrusion having a surface that faces the first surface of the flexure element, the protrusion protruding toward the first surface of the flexure element beyond the recess. A gap is formed between the recess and the first surface of the flexure element, and the pulse wave sensor is configured to detect a pulse wave based on a detection value of the detection element.

## Description

### TECHNICAL FIELD

The present invention relates to a pulse wave sensor.

### BACKGROUND

Pulse wave sensors that detect pulse waves generated when a heart pumps blood are known. One example is a pulse wave sensor including a flexure element that is flexibly supported so as to be bendable by the action of an external force; and a detection element that detects strain in the flexure element or pressure applied to the flexure element.

For example, Patent Document 1 discloses a pulse wave sensor that includes the flexure element and a strain gauge. In the pulse wave sensor, the strain gauge that detects strain of the flexure element is provided on a bottom surface of the flexure element. A top surface of the flexure element is pressed against the skin of a measurement subject. In this case, the strain of the flexure element that is subjected to minute pressure from pulse waves can be detected by the flexure element.

### RELATED-ART DOCUMENTS

### PATENT DOCUMENTS

Patent Document 1: Japanese Unexamined Patent Application Publication No. 2022-175355

### SUMMARY

### PROBLEM TO BE SOLVED BY THE INVENTION

In order to obtain biological signal waves using the above pulse wave sensor that detects the strain of the flexure element, it is necessary to reliably identify the position of the radial artery or ulnar artery of a biological subject to be measured, and to bring the flexure element into contact with the identified position with appropriate pressure.

That is, in conventional pulse wave sensors, users of the pulse wave sensors need to identify the position of the radial artery or ulnar artery, and then press the pulse wave sensor against either of these arteries at both an appropriate angle and an appropriate pressure. When these requirements are not met, it may take time to obtain the biological signal waves or errors in obtaining the biological signal waves may occur.

An object of the present invention is to provide a pulse wave sensor that can be more easily usable.

### MEANS FOR SOLVING THE PROBLEM

A pulse wave sensor according to one embodiment of the present disclosure is a pulse wave sensor to be used in contact with a body of a measurement target. The pulse wave sensor includes a flexure element having a first surface that is on a measurement target side, and a second surface located opposite to the first surface; at least one detection element disposed on the second surface, and configured to detect strain of the flexure element and/or pressure applied to the flexure element; and a cover member mounted or formed on the first surface. The cover member includes a recess opening toward the first surface of the flexure element, and a protrusion having a surface that faces the first surface of the flexure element, the protrusion protruding toward the first surface of the flexure element beyond the recess. A gap is formed between the recess and the first surface of the flexure element, and the pulse wave sensor is configured to detect a pulse wave based on a detection value of the detection element.

### EFFECTS OF THE INVENTION

According to a disclosed technology, a pulse wave sensor which can be more easily usable can be implemented.

### BRIEF DESCRIPTION OF THE DRAWINGS

[FIG. 1] FIG. 1 is a perspective view illustrating a pulse wave sensor according to a first embodiment.
[FIG. 2] FIG. 2 is a cross-sectional view illustrating the pulse wave sensor according to the first embodiment.
[FIG. 3] FIG. 3 is a diagram illustrating an example of a bridge circuit.
[FIG. 4] FIG. 4 is a cross-sectional view illustrating the pulse wave sensor in a first modification of the first embodiment.
[FIG. 5] FIG. 5 is a perspective view illustrating a flexure element and a cover member of the pulse wave sensor in the first modification of the first embodiment.
[FIG. 6] FIG. 6 is a cross-sectional view illustrating the pulse wave sensor in a second modification of the first embodiment.
[FIG. 7] FIG. 7 is a cross-sectional view illustrating the pulse wave sensor in a third modification of the first embodiment.
[FIG. 8] FIG. 8 is a cross-sectional view illustrating the pulse wave sensor in a fourth modification of the first embodiment.
[FIG. 9] FIG. 9 is a graph illustrating measurement results of a normalized average pulse wave and a normalized average acceleration pulse wave.
[FIG. 10] FIG. 10 is a graph illustrating measurement results of the normalized average pulse wave.
[FIG. 11] FIG. 11 is a graph illustrating a strain magnitude when a load is applied to a pulse wave sensor without the cover member.
[FIG. 12] FIG. 12 is a graph illustrating the strain magnitude when the load is applied to the pulse wave sensor with the cover member.
[FIG. 13] FIG. 13 is a graph illustrating the pulse wave sensor applied diagonally to a measurement target.
[FIG. 14] FIG. 14 is a cross-sectional view illustrating a strain gauge according to the first embodiment.
[FIG. 15] FIG. 15 is a cross-sectional view (part 1) illustrating the strain gauge according to the first embodiment.
[FIG. 16] FIG. 16 is a cross-sectional view (part 2) illustrating the strain gauge according to the first embodiment.

### MODE FOR CARRYING OUT THE INVENTION

Embodiments of the present invention will be described below with reference to the drawings. In each of the drawings, the same components are denoted by the same reference numerals, and duplicate description may be omitted.

A pulse wave sensor in the present disclosure is a pulse wave sensor to be used in contact with a body of a measurement target. The pulse wave sensor includes a flexure element having a first surface that is on a measurement target side, and a second surface located opposite to the first surface; at least one detection element disposed on the second surface, and configured to detect strain of the flexure element and/or pressure applied to the flexure element; and a cover member mounted or formed on the first surface. The pulse wave sensor is configured to detect a pulse wave based on a detection value of the detection element. In a pulse wave sensor in the present disclosure, a cover member includes a recess opening toward the first surface of the flexure element, and a protrusion having a surface that faces the first surface of the flexure element, the protrusion protruding toward the first surface of the flexure element beyond the recess. A gap is formed between the recess and the first surface of the flexure element. As the detection element, for example, a strain gauge or a piezoelectric element can be used.

### <First Embodiment>

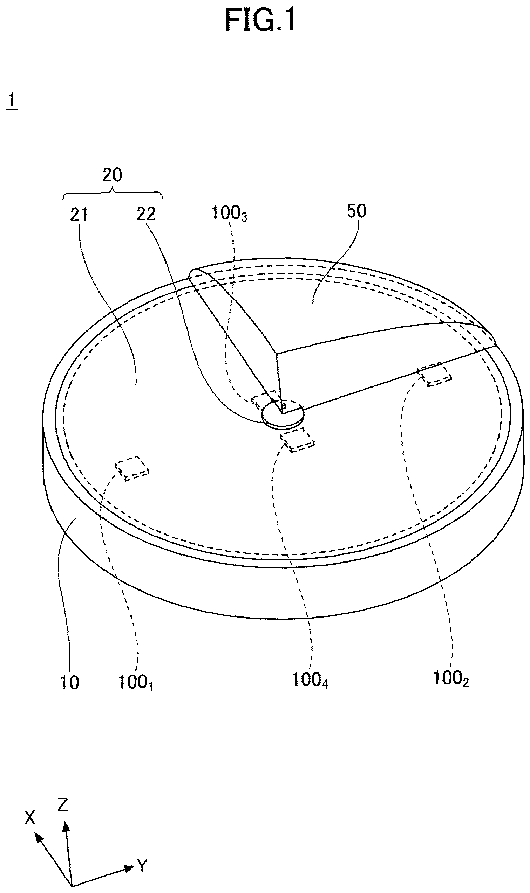

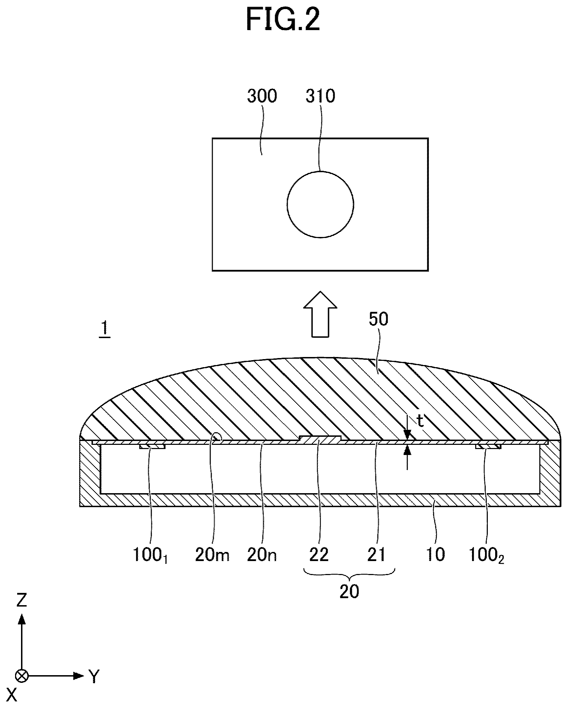

In a first embodiment, a pulse wave sensor 1 will be described as an example of a pulse wave sensor in the present disclosure. FIG. 1 is a perspective view illustrating the pulse wave sensor according to the first embodiment. FIG. 2 is a cross-sectional view illustrating the pulse wave sensor according to the first embodiment, and illustrating a longitudinal section of the pulse wave sensor 1 passing through the centers of strain gauges 100₁ and 100₂ shown in FIG. 1. In FIG. 1, a cover member 50 illustrates a cut cross-sectional view. FIG. 2 schematically illustrates a body of a measurement target (300) and an artery 310 inside the body.

Referring to FIGS. 1 and 2, the pulse wave sensor 1 has a housing 10, a flexure element 20, a cover member 50, and a plurality of strain gauges (100₁, 100₂, 100₃, and 100₄). When there is no particular need to distinguish among these strain gauges, the strain gauges may be collectively referred to as strain gauges 100.

The housing 10 is a portion that holds the flexure element 20. The housing 10 has a hollow cylindrical shape, for example, and a lower surface side of the housing 10 is closed and an upper surface side of the housing 10 is open. The housing 10 can be made of, for example, a metal or resin. The flexure element 20 that is substantially disk-shaped is fixed by adhesive or the like so as to close an opening on the upper surface side of the housing 10.

The flexure element 20 directly or indirectly contacts the artery 310 of the measurement target 300. The flexure element 20 is plate-shaped. The flexure element 20 has a first surface 20m on a measurement target side and a second surface 20n on an opposite side of the first surface 20m. The flexure element 20 has a base 21 and a load portion 22. The flexure element 20 may have a configuration with a plurality of load portions 22. The flexure element 20 may have a configuration without a load portion 22.

In the description of FIGS. 1 to 13, as shown in FIG. 2, when the pulse wave sensor 1 is vertically applied to the measurement target, a side of the cover member 50 or the flexure element 20 that contacts the measurement target is referred to as an "upper side," and a side opposite to the upper side is referred to as a "lower side." In addition, a surface positioned on the upper side of each portion is referred to as the "upper side," and a surface positioned on the lower side of each portion is referred to as a "lower side." The pulse wave sensor 1 can be applied to the measurement target at any angle, not only vertically as described above. A plan view refers to viewing the target in the same direction as the normal from the upper side to the lower side with respect to the upper surface (i.e., a first surface 20m) of the flexure element 20. A planar shape refers to a shape of the target when viewing the target in a normal direction.

In the flexure element 20, a base 21 is circular in plan view, for example. The diameter of the base 21 is, for example, greater than or equal to 10 mm and less than or equal to 15 mm.

The load portion 22 is provided on the base 21. The load portion 22 has a circular shape having a diameter less than that of the base 21 in plan view, for example. In the present embodiment, the load portion 22 is cylindrical, but the load portion 22 may be formed as a curved surface protruding from the base 21, for example. The load portion 22 is arranged such that the center of the load portion 22 coincides with the center of the base 21 in plan view, for example. The load portion 22 protrudes from the upper surface (that is, the first surface 20m of the flexure element 20) of the base 21. When the load portion 22 is provided, the pressure propagating to the pulse wave sensor 1 can be concentrated on the load portion 22 in a case where the pulse wave sensor 1 is applied to the measurement target 300. A protrusion amount of the load portion 22 with respect to the upper surface of the base 21 is about 0.1 mm, for example. The base 21 is flexible and elastically deforms when a load is applied to the base 21 directly or indirectly.

In the pulse wave sensor 1, the material of the flexure element 20 is not particularly limited. The flexure element 20 may be made of, for example, metal, glass, ceramic, plastic, or the like. The flexure element 20 may or may not have a slit. When the slit is provided, a strain magnitude of the flexure element can be increased. When the slit is not provided, the fracture strength of the flexure element 20 can be increased.

A thickness t of the flexure element 20 excluding the load portion 22 may be constant. The thickness t can be, for example, greater than or equal to 0.03 mm and less than or equal to 0.3 mm. If the thickness t is 0.03 mm or more, the required fracture strength can be obtained. If the thickness t is 0.3 mm or less, it can be easily deformed by external loads.

When the flexure element 20 is made of metal, examples of metals used as the material of the flexure element 20 include SUS (stainless steel), copper, aluminum, and the like. The flexure element 20 can be integrally formed by, for example, etching, pressing, or the like.

When the flexure element 20 is made of glass, the glass used for the flexure element 20 may be unreinforced glass or reinforced glass. The reinforced glass may be chemically or physically reinforced glass. The chemically reinforced glass is preferable in terms of being able to reinforce even glass with a thin plate. Since the chemically reinforced glass can increase the compressive stress on its surface, the fracture strength of the chemically reinforced glass is higher than that of unreinforced glass.

When the flexure element 20 is made of glass, the thickness t may be greater than or equal to that of a flexure element made of metal, for example. When the flexure element 20 is made of glass, the load portion 22 can be formed, for example, by etching one side of the glass that has a constant thickness.

The Young's modulus of glass is, for example, greater than or equal to 60 GPa and less than or equal to 90 GPa. On the other hand, the Young's modulus of stainless steel (SUS), which is preferably used for the flexure element that is made of metal, is about 210 GPa. In this arrangement, the glass is a material having a relatively low Young's modulus. In this case, the flexure element made of glass deforms more easily than the flexure element made of metal if these flexure elements have the same shape (that is, easily deformed) . Therefore, when producing a glass flexure element with the same shape as a metal flexure element, strain due to pulse waves can be detected with higher sensitivity. When producing a glass flexure element having the same sensitivity as the metal flexure element, the glass flexure element can be made thicker, the load portion can be made smaller (or the load portion can be eliminated). In other words, design flexibility of the flexure element is increased.

The glass is a material that is unlikely to undergo plastic deformation. For this reason, in the pulse wave sensor 1 that employs the flexure element 20 made of glass, plastic deformation over time and sudden plastic deformation of the flexure element 20 can be suppressed compared with a case where the metal flexure element is employed. Furthermore, because the glass can be made into reinforced glass as described above, the fracture strength can be made higher than that of metal, depending on the type of reinforcement. As a result, the reinforced glass flexure element can further suppress the plastic deformation over time and the sudden plastic deformation.

In this arrangement, when the glass flexure element 20 is employed in the pulse wave sensor 1, the stability of detection sensitivity for a biological signal can be improved. As a result, it is possible to obtain more accurate values with reduced errors for various health and/or medical-related parameters (for example, pulse wave(s)) identified based on the biological signal. The biological signal refers to a pulse wave and/or intermediate parameter(s) used to identify the pulse wave.

In addition, by adopting a glass flexure element 20 with relatively low Young's modulus, the pulse wave sensor 1 can detect the strain (or pressure) of the flexure element 20 with more sensitivity than a conventional flexure element (for example, a flexure element made of metal). That is, by adopting the glass flexure element 20, the pulse wave sensor 1 that can detect the biological signal with high sensitivity can be implemented.

The size, shape, and thickness of the flexure element 20 may be determined such that the fracture strength under static pressure is greater than 20 N. With such fracture strength, plastic deformation over time and sudden plastic deformation of the flexure element 20 can be further reduced. In addition, the flexure element 20 may have a structure without a slit, or a structure in which the strain gauge 100 is arranged on a beam having slits that is divided by the slits. When the flexure element 20 is provided with a slit, the strain magnitude of the flexure element 20 can be increased. When the flexure element 20 is not provided with a slit, the fracture strength of the flexure element 20 can be increased.

An output signal of the pulse wave sensor 1 is generated based on the outputs of a plurality of strain gauges. In the illustrated example, on a second surface 20n of the flexure element 20, the pulse wave sensor 1 has a pair of strain gauges 100₁ and 100₂ arranged on opposite sides of the load portion 22 in a Y direction in plan view. In the plan view, the pulse wave sensor 1 has the other pair of strain gauges 100₃ and 100₄ that are arranged on opposite sides of the load portion 22 in an X direction.

Each strain gauge 100 is a detection element that detects the strain of the flexure element 20. The strain gauges 100₁ and 100₂ detect compressive strain of the flexure element 20 that is caused by pressing on the load portion 22. The strain gauges 100₃ and 100₄ detect tensile strain of the flexure element 20 that is caused by pressing the load portion 22. A distance between the strain gauge 100₁ and the strain gauge 100₂ that detect the compressive strain is larger than a distance between the strain gauge 100₃ and the strain gauge 100₄ that detect the tensile strain. By arranging strain gauges 100 as described above, it is possible to effectively detect the compressive strain and the tensile strain to thereby obtain a large output by a bridge circuit that constitutes a full bridge.

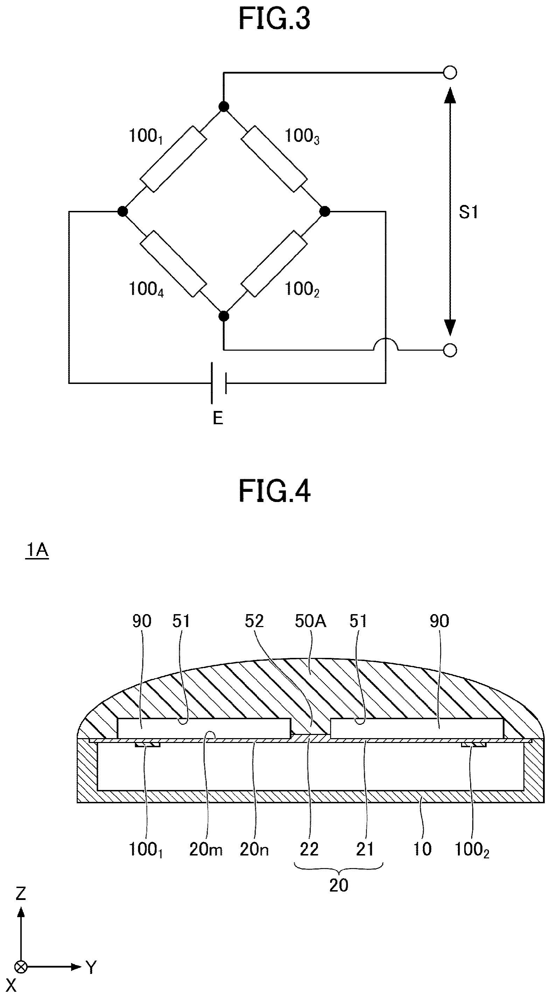

The strain gauges 100₁ to 100₄ are connected so as to constitute respective sides of one bridge circuit, and an output signal of the pulse wave sensor 1 can be generated by the bridge circuit. FIG. 3 is a diagram illustrating an example of the bridge circuit. In the bridge circuit shown in FIG. 3, the strain gauge 100₁ constitutes an upper left side. The strain gauge 100₂ constitutes a lower right side. The strain gauge 100₃ constitutes an upper right side. The strain gauge 100₄ constitutes a lower left side.

In FIG. 3, a DC voltage E is supplied between a node connecting the upper left side and the lower left side, and a node connecting the upper right side and the lower right side. In this arrangement, an analog voltage output signal S1 can be obtained from between the node connecting the upper left side and the upper right side, and the node connecting the lower left side and the lower right side. The bridge circuit can be provided, for example, on a circuit board attached to an inner surface of the housing 10.

In the pulse wave sensor 1, when the load portion 22 contacts the radial artery to be measured, a load is applied to the load portion 22 according to a pulse wave to be measured, the flexure element 20 elastically deforms, and resistance values of one or more resistors of the strain gauge 100 change. The pulse wave sensor 1 can detect the pulse wave based on changes in resistance values of one or more resistors of the strain gauge 100 due to deformation of the flexure element 20. The pulse wave is detected as the output signal S1 representing a change in a periodic voltage from the bridge circuit.

In the above example, the pulse wave sensor 1 has four strain gauges (strain gauges 100₁ to 100₄), and the output signal S1 is generated by connecting the four strain gauges in a full-bridge arrangement. However, the pulse wave sensor 1 may have two strain gauges having the same configuration as the strain gauges 100₁ to 100₄, and the bridge circuit may generate the output signal S1 by connecting the two strain gauges in a half bridge arrangement. Specific structure examples of the strain gauges will be described later.

The pulse wave sensor 1 may have a cable, a shielded cable, a flexible substrate, or the like. Without using a cable or the like, the pulse wave sensor 1 may have a configuration that communicates with an external circuit or device wirelessly or a similar connection.

The cover member 50 is mounted on the flexure element 20 so as to cover the first surface 20m of the flexure element 20. The shape of the cover member is not particularly limited, but it is preferable that the cover member 50 be designed such that, for example, when the skin to be measured is pressed against the first surface 20m of the flexure element 20 at a non-perpendicular angle, pressure is applied relatively evenly to the load portion 22. Furthermore, it is desirable that the cover member 50 be designed such that the pressure propagated from the measurement target 300 is applied relatively evenly even when a contact condition between the measurement target and the cover member 50 (that is, the magnitude of a pressing force that presses when pressing the cover member 50 against the measurement target 300, which external part of the cover member 50 is in contact with the measurement target 300, and an angle at which the cover member 50 is pressed against the measurement target 300) changes.

As a preferable example of the above "shape such that the pressure is applied relatively evenly," a hemispherical cover member 50 as shown in FIG. 1 and FIG. 2 is used. As described above, the upper surface of the cover member 50 can be a curved surface that is highest at a central portion of the first surface 20m of the flexure element 20 and gradually decreases toward a peripheral portion of the first surface 20m of the flexure element 20, with reference to the first surface 20m of the flexure element 20.

As another example of the shape of the cover member 50, for example, a shape having surfaces including rotational conic sections, such as those represented by (A) a spheroid of revolution (semi-ellipsoid), (B) a rotational super-ellipsoid (rotational oval ellipsoid), (C) an ovoid shape, and (D) a shape having a paraboloid of revolution, is used. For example, the upper surface of the cover member 50 may be a surface including such a rotational conic section.

The cover member 50 is not limited to (A) to (D) above, and may have a shape having a surface including a spline curve approximating a rotational conic section (i.e., a spline surface), or a shape having a surface approximating the rotational conic section by a straight line and a curve. More broadly defined, the cover member 50 may have a shape such that a perpendicular line at any point on the upper surface (that is, a surface portion of the cover member 50 that contacts the skin to be measured) of the cover member 50 intersects with a perpendicular line of the first surface 20m that passes through the center of the first surface 20m of the flexure element 20.

As described above, the structure of the cover member is determined in consideration of (i) the mechanical relationship determined by the shape of the surface (a surface contacting the measurement target) and the shapes of the bottom surface (the surface mounted on the flexure element) and a gap, and (ii) physical properties determined by the material of the cover member (for example, hardness and elastic modulus).

The cover member 50 is, for example, made of silicone. A method of mounting or forming the cover member 50 in the pulse wave sensor 1 is not particularly limited. For example, the cover member 50 may be formed separately from other members of the pulse wave sensor 1 and fitted into the housing 10. That is, the cover member 50 may be a cap-like member that covers the flexure element 20. For example, the cover member 50 may be formed by inserting silicone into the first surface 20m of the flexure element 20. Film-shaped silicone may be bonded to the first surface 20m as the cover member 50. The material of the cover member 50 is not limited to the silicone. For example, the cover member 50 may be a thermosetting resin such as polyurethane resin, epoxy resin, or phenol resin, or, a thermoplastic resin such as polyethylene resin, polypropylene resin, polyvinyl chloride resin, polystyrene resin, ABS resin, acrylic resin, or polycarbonate resin. Other materials may be used as long as these materials have a combination of shapes and physical properties that can achieve a function of the cover member 50 as described above.

As described above, by mounting the cover member 50 on the first surface 20m of the flexure element 20, the pressure applied to the cover member 50 can be concentrated on the load portion 22 of the flexure element 20. In this arrangement, a measurable range of pressure can be expanded, and detection accuracy can be improved. That is, in the pulse wave sensor 1, during use, it is easy to identify the position of either the radial artery or ulnar artery, and further, to press the flexure element 20 against either of these arteries at both appropriate angle and an appropriate pressure. As a result, a pulse wave sensor in which the time required to acquire the biological signal wave can be reduced, in which acquisition errors of the biological signal wave is unlikely to occur, and that is easier to use can be realized. Here, "expansion of the measurable range" means that when the pulse wave sensor is applied to the skin (for example, wrist) above the artery to be measured, a range of pulse waves that can be properly measured expands.

In addition, when the flexure element 20 is made of metal, by providing the cover member 50, the flexure element 20 made of metal does not directly contact the skin of a subject to be measured. Thus, skin inflammation and metal allergy development in the subject due to the metal can be avoided. In addition, when the flexure element 20 is made of glass, by providing the cover member 50, scattering of fragments can be prevented even if the flexure element 20 cracks.

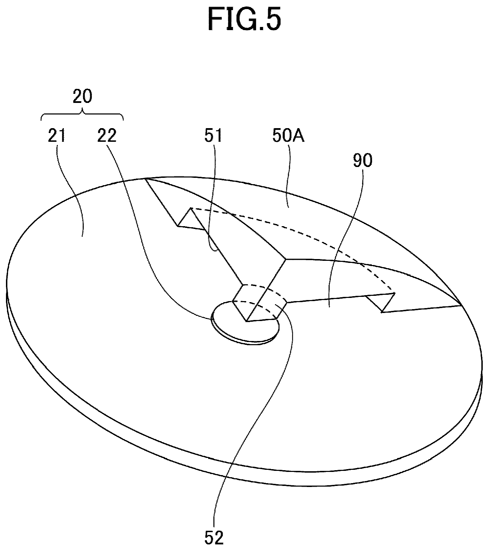

FIG. 4 is a cross-sectional view illustrating the pulse wave sensor in a first modification of the first embodiment. FIG. 5 is a perspective view illustrating the flexure element and the cover member of the pulse wave sensor in the first modification of the first embodiment. A pulse wave sensor 1A shown in FIGS. 4 and 5 is equipped with a cover member 50A. The cover member 50A has an upper surface as the exterior that contacts a measurement target. The cover member 50A has a recess 51 that opens toward a first surface side (inner side) of the first surface 20m of the flexure element 20. A gap 90 (clearance) is formed between the recess 51 and the first surface 20m of the flexure element 20. As a result, it is preferable to provide the gap 90 between the cover member 50A and the flexure element 20.

The cover member 50A may have a protrusion 52 that protrudes further toward the first surface 20m than the recess 51, at a central portion in plan view. The protrusion 52 may be provided so as to face the load portion 22. That is, the protrusion 52 and the load portion 22 may overlap in plan view. The recess 51 is formed in a ring shape so as to surround the protrusion 52 in plan view, for example. That is, the gap 90 is formed to have a ring shape so as to surround the protrusion 52 in plan view, for example. The protrusion 52 may be a protrusion formed by planar surfaces as shown in FIGS. 4 and 5, or may be a protrusion formed by a curved surface.

The protrusion 52 of the cover member 50A may be designed to contact the load portion 22 (if the load portion 22 is not provided, the base 21 of the flexure element 20 is used) of the flexure element 20 when no pressure is applied to the cover member 50A. In this case, the surface or point where the protrusion 52 and the load portion 22 (or the base 21) are in contact may be bonded or may not be bonded.

Alternatively, the protrusion 52 of the cover member 50A may be designed to have a gap between the protrusion 52 and the load portion 22 (if the load portion 22 is not provided, the base 21 is used) when no pressure is applied to the cover member 50A. That is, the protrusion 52 and the flexure element 20 (or the load portion 22) may be separated when no pressure is applied. Regardless of the presence or absence of the gap, the protrusion 52 and the load portion 22 (or the base 21) are designed such that, when the cover member 50A is pressed against a body to be measured, the protrusion 52 contacts the load portion 22 (or the base 21) and transmits pressure.

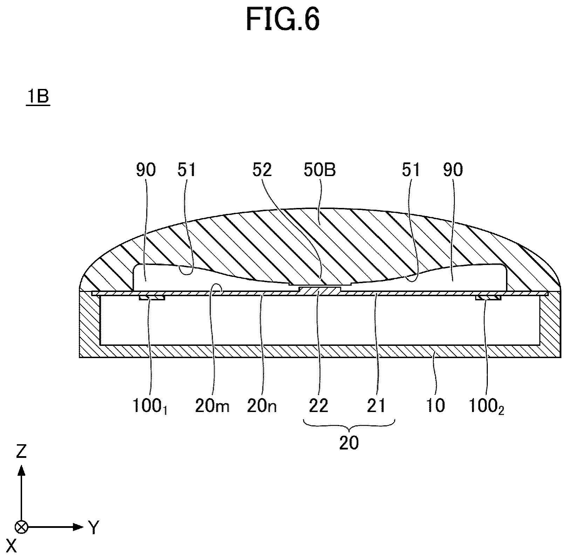

FIG. 6 is a cross-sectional view illustrating the pulse wave sensor in a second modification of the first embodiment. A pulse wave sensor 1B shown in FIG. 6 is equipped with a cover member 50B. In the pulse wave sensor 1A shown in FIG. 5, the recess 51 that forms the gap 90 does not include an inclined curved surface, but in the pulse wave sensor 1B shown in FIG. 6, the recess 51 that forms the gap 90 includes an inclined curved surface. In the pulse wave sensor 1B, the inclined curved surface of the recess 51 is a continuous curved surface, but the surface need not be the continuous curved surface. For example, the inclined curved surface may have a shape approximating a curved surface, where flat surfaces are connected in fragments.

By designing cover members such as the pulse wave sensors 1A and 1B to have the protrusions 52, the pressure applied to the cover members can be concentrated on the protrusions 52 even when the cover member is improperly applied to the measurement target. Here, "when the cover member is improperly applied to the measurement target" means, for example, when the cover member is applied to the skin (for example, wrist) of a measurement target, the cover member is applied at a slight angle rather than in a direction perpendicular to the first surface 20m of the flexure element 20, or the cover member is applied at a position slightly offset from directly above the artery. Even in such a case, the pressure can be concentrated on the protrusion 52 according to each of the pulse wave sensors 1A and 1B. As a result, a pressure measurable range can be expanded according to each of the pulse wave sensors 1A and 1B. Moreover, even in a case where the cover member is improperly applied to the measurement target as described above, a pressure distribution similar to that obtained when properly applied can be achieved, and thus detection accuracy of the biological signal can be stabilized according to the pulse wave sensors 1A and 1B.

The shape and size of the gap 90 itself are not particularly limited if the gap is designed so that pressure is most concentrated on the protrusion 52.

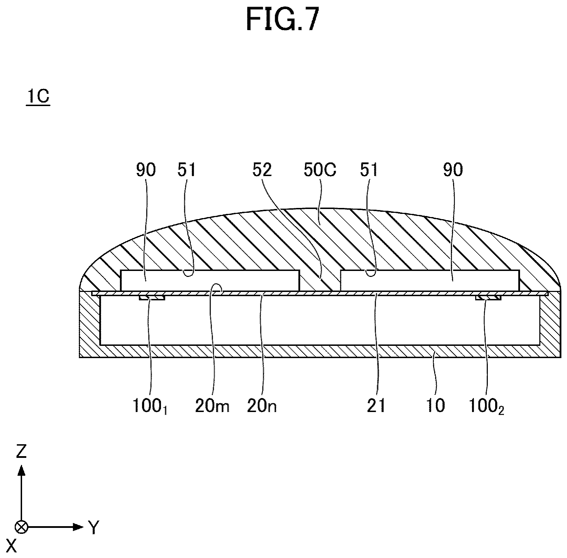

FIG. 7 is a cross-sectional view illustrating the pulse wave sensor in a third modification of the first embodiment. A pulse wave sensor 1C shown in FIG. 7 is equipped with a cover member 50C. Like the pulse wave sensor 1C, the pulse wave sensor in the present disclosure may have a configuration in which the load portion 22 is not provided in the flexure element 20 and the protrusion 52 of the cover member 50C is in contact with the first surface 20m of the flexure element 20. Alternatively, the pulse wave sensor in the present disclosure may have a configuration in which the protrusion 52 is in contact with the first surface 20m of the flexure element 20 when pressure is applied to the cover member 50C. In either case, the same effect as in a case where the load portion 22 is provided can be obtained. When the protrusion 52 and the flexure element 20 are in contact with each other in the pulse wave sensor, the contacting surface or point may be bonded.

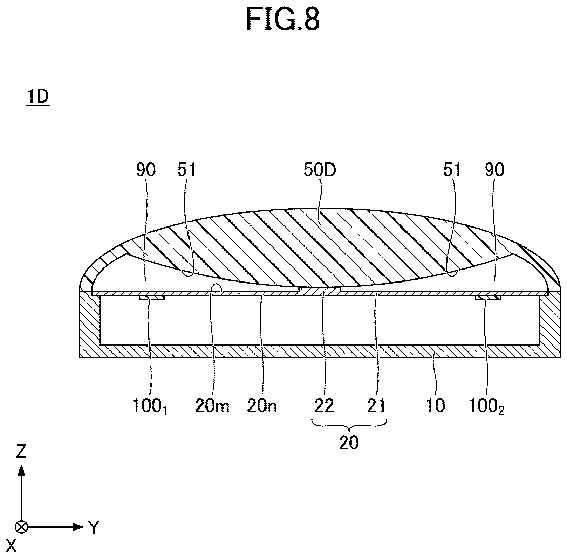

FIG. 8 is a cross-sectional view illustrating the pulse wave sensor in a fourth modification of the first embodiment. A pulse wave sensor 1D shown in FIG. 8 is equipped with a cover member 50D. The cover member 50D differs from the cover member 50A and the like in that there is no clearly protruding portion such as the protrusion 52 on a surface facing the flexure element 20. For other aspects of the cover member 50D, the configuration may be similar to that of the cover member 50A and the like, or modifications similar to those applied to the cover members 50A, 50B, and 50C may be made.

The cover member 50D has a curved surface in which at least a portion of the surface on a side facing the flexure element 20 curves toward the flexure element 20. In the pulse wave sensor 1D in the present modification, the entire curved surface of the cover member 50D serves as a protrusion. In an example of FIG. 8, the cover member 50D has a curved surface in which a central portion of the surface facing the flexure element 20 gently slopes toward the flexure element 20. With this design, the pressure applied to the cover member 50D can be concentrated on a portion (for example, the load portion 22 of the flexure element 20) where the cover member 50D and the flexure element 20 are in contact. As a result, with use of the cover member 50D, the pulse wave sensor 1D can concentrate stress within a specific region of the flexure element 20.

Similarly to the cover member 50, the shape of the cover members 50A, 50B, 50C, and 50D is not limited to a hemispherical shape. For example, as provided in the description of the cover member 50, the cover members 50A, 50B, 50C, and 50D may have a shape having a surface including a rotation conic section, a shape having a spline curved surface, or a shape having a surface approximating the surface including the rotation conic section by a straight line and a curve.

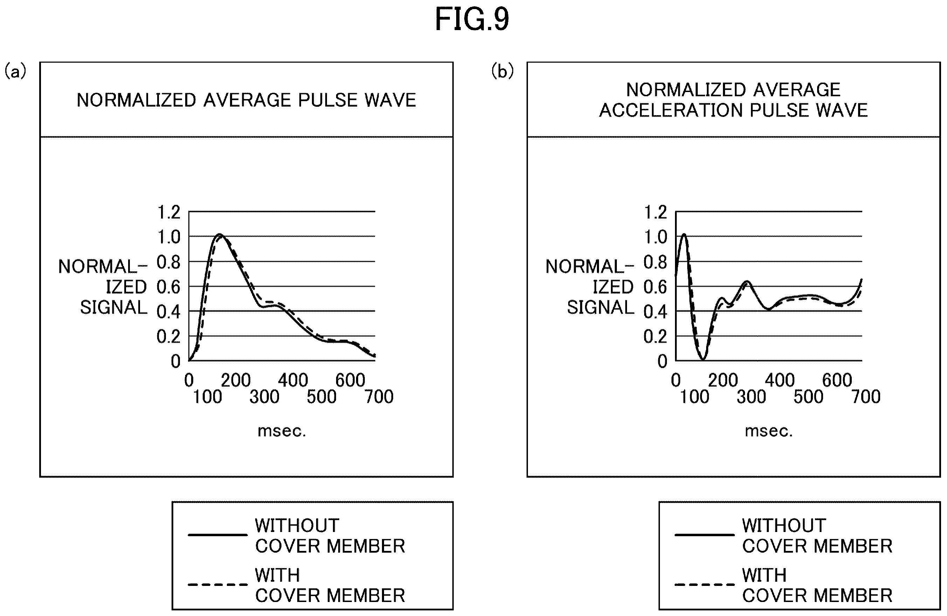

Hereinafter, the effects of the pulse wave sensor in the present disclosure will be described with reference to FIGS. 9 to 12. However, the pulse wave sensor in the present disclosure is not limited to the configuration illustrated in FIGS. 9 to 12.

FIG. 9 is a graph showing the measurement results of normalized average pulse waves and normalized average acceleration pulse waves with and without the cover member attached to the pulse wave sensor. A metal flexure element is used as a flexure element of the pulse wave sensor. Since the pulse wave sensor may have different pressing forces for each measurement, an output level voltage changes. For this reason, normalization is performed based on the maximum value and minimum value of the output. The vertical axis of the graph represents the normalization of the biological signal wave. The horizontal axis of the graph represents the time from a minimum value of the pulse wave (namely, during vascular contraction).

FIG. 9(a) is a graph showing the normalized average pulse wave and FIG. 9(b) is a graph showing the normalized average acceleration pulse wave. In these two graphs, the "without cover member" curve indicates a measurement result in a state where the cover member is not attached to the pulse wave sensor, and the "with cover member" curve indicates a measurement result in a state where the cover member made of silicone as shown in FIG. 2 is attached to the same pulse wave sensor as in the "without cover member" curve. The measurement is performed in a state where the pulse wave sensor is applied to the measurement target at an appropriate position and angle. A case where "the pulse wave sensor is applied to the measurement target at the appropriate position and angle" means that the pulse wave sensor is applied at a position directly above the artery 310 of the measurement target 300 and at a substantially perpendicular angle to the measurement target 300, as shown in FIG. 2.

As shown in FIG. 9(a) and (b), with metal flexure elements, the average pulse wave and the average acceleration pulse wave show approximately equivalent values regardless of whether the cover member is attached. In other words, even if the metal flexure element is covered with the cover member, measurement accuracy of the pulse wave is the same as that in a case where the cover member is not covered. In other words, the measurement accuracy does not decrease due to the presence of the cover member.

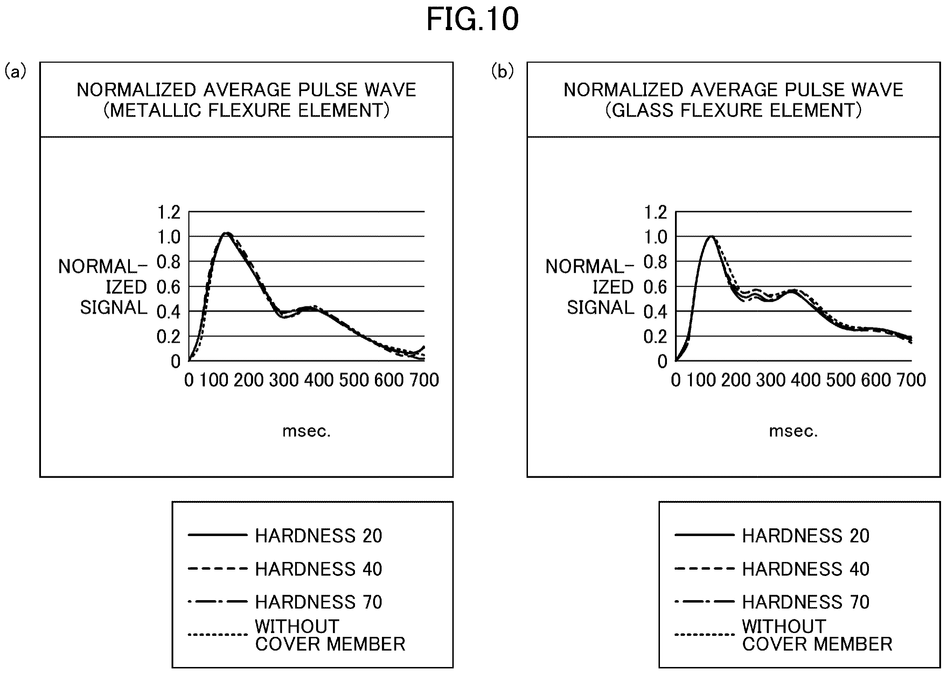

FIG. 10 is a graph showing the measurement results of normalized mean pulse waves when cover members of various hardness are attached to a metal flexure element and a glass flexure element. The measurement is performed with the pulse wave sensor that is applied at an appropriate position and angle. The vertical and horizontal axes of Fig. 10(a) and (b), as in Fig. 9(a), represent the normalized biological signal wave on the vertical axis. The horizontal axis represents the time from the minimum value of the pulse wave. The graph in Fig. 10(a) shows the measurement results when using a metal flexure element, and the graph in Fig. 10(b) shows the measurement results when using a glass flexure element. Various lines in these two graphs show the measurement results when the cover member has a Shore A hardness of 20, 40, and 70, respectively. In Fig. 10(a) and (b), a silicone cover member having the shape shown in Fig. 6 is used. The measurement results without the cover member are also shown.

As shown in FIG. 10, in the case of the metal flexure element, the substantially similar measurement results are obtained regardless of the presence or absence of the cover member and the hardness of the cover member. In the case of the glass flexure element, the substantially similar measurement results are obtained regardless of the presence or absence of the cover member and the hardness of the cover member. Therefore, it can be said that measurement accuracy does not decrease due to the presence of the cover member, whether the flexure element is made of metal or glass. Moreover, that the hardness of the cover member has little to no effect on measurement accuracy, as long as the Shore A hardness is at least Shore 20 to 70.

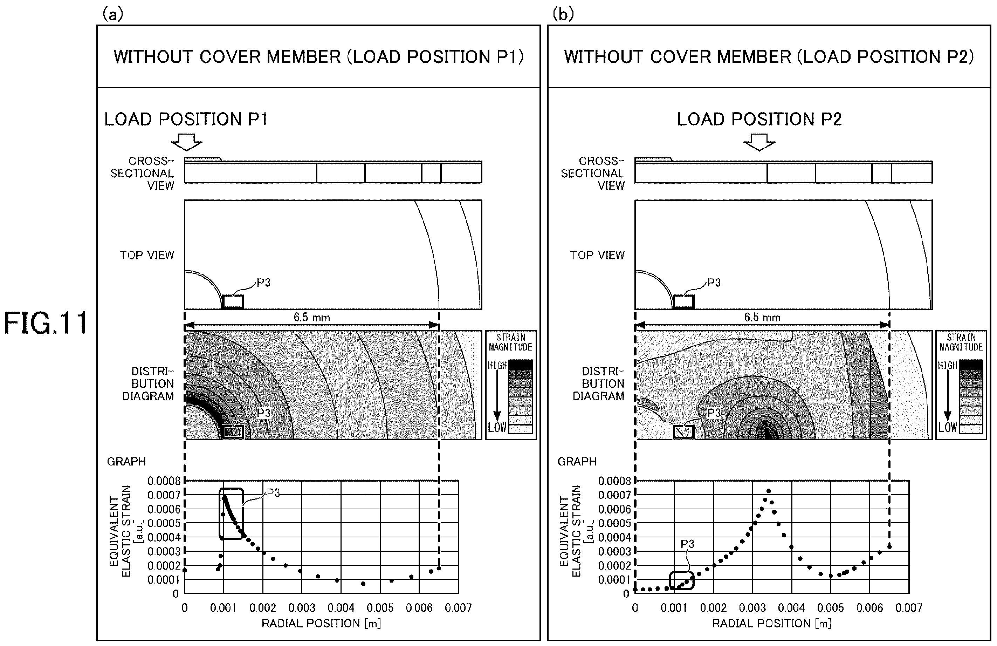

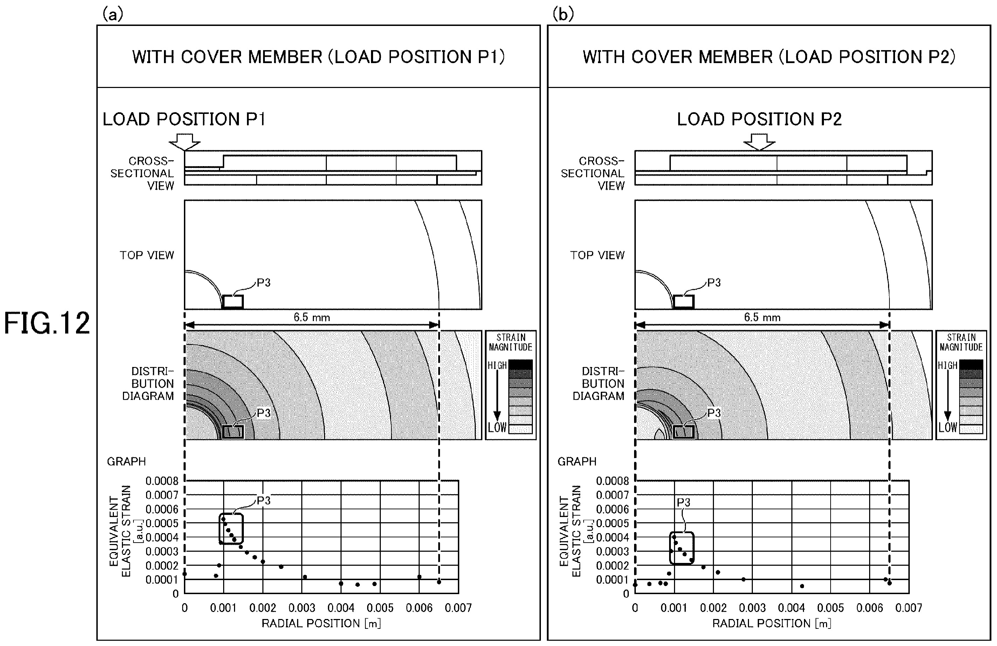

Hereinafter, the difference in the strain distribution in the flexure element with and without the cover member is shown using FIGS. 11 and 12. FIGS. 11 and 12 illustrates the results of numerical simulations of a state of the flexure element when loads are applied to pulse wave sensors having predetermined configurations, respectively.

FIG. 11 is a diagram showing the strain magnitude when the load is applied to a pulse wave sensor without the cover member. The configuration of the pulse wave sensor shown in FIG. 11 is the same as that of the cover member 50 from which the pulse wave sensor 1 is removed. A series of drawings shown in FIG. 11(a) are drawings when a predetermined load is applied to a load position P1 around the load portion. On the other hand, a series of drawings shown in FIG. 11(b) shows a case where a load of the same magnitude as the load applied to the load position P1 is applied at a load position P2, which is located slightly away from the load position in a radial direction. FIGS. 11(a) and (b) each illustrate four figures: a cross-sectional view, a top view, a distribution diagram, and a graph. The horizontal axis is common to all figures, and shows the distance from the center of the flexure element. In other words, these four figures are arranged vertically with the distance from the center of the flexure element aligned. The load position (P1) is common across the four figures of FIG. 11(a). The load position (P2) is common across the four figures of FIG. 11(b). The following describes how to interpret each figure in FIG. 11.

The "cross-sectional views" in FIG. 11(a) and (b) are cross-sectional views of the flexure elements taken along straight lines passing through the centers of the flexure elements. White arrows in these figures indicate the positions where the loads are applied to the flexure elements (load position P1 and P2), respectively. FIGS. 11(a) and (b) show the "top views" of a part of the flexure element in plan view and attachment positions P3 of the strain gauges. As described above, each strain gauge is actually attached to the lower surface of the flexure element.

The "distribution diagrams" in FIGS. 11(a) and (b) show maps of strain magnitudes of the flexure elements when predetermined loads are applied to the load positions P1 and P2 of the flexure elements as shown in the "top views." Here, the "strain magnitude" refers to a magnitude of an "equivalent strain" derived from both tensile and compressive strains. The strain magnitude is indicated by a hatching pattern, and a legend is appended. The "graphs" in FIGS. 11(a) and (b) are graphs where the vertical axes represent the strain magnitudes shown in the distribution diagrams and the horizontal axes represent the distances from the centers of the flexure elements. In the distribution diagrams and the graphs, each position P3 where the strain gauge is attached is indicated by a box in the figure as in the top view.

Similarly to FIG. 11, FIG. 12 shows the strain magnitude when the cover member is attached to the pulse wave sensor and a load is applied. The cover member in an example shown in FIG. 12 has the same shape as the cover member 50A shown in FIG. 4. The difference between (a) of FIG. 12 and (b) of FIG. 12, and the way of reading the cross-sectional view, top view, distribution diagram, and graph are the same as those in FIG. 11(a) and (b). However, FIG. 12(a) and (b) illustrate the schematically cross-sectional views of the cover members, and the illustration of the cover members is omitted in the top views. The same load position P1 is indicated in FIG. 11 and FIG. 12. Similarly, the same load position P2 is indicated in FIG. 11 and FIG. 12. Furthermore, the same strain gauge attachment position P3 is indicated in FIG. 11 and FIG. 12.

Referring to the distribution diagrams and graphs shown in FIG. 11(a) and (b), when pressure is applied to the load position P1 of the flexure element, strain is concentrated in the vicinity of the load position P1 (FIG. 11(a)), and when pressure is applied to the load position P2, strain is concentrated in the vicinity of the load position P2 (FIG. 11(b)). On the other hand, referring to the distribution diagrams and graphs shown in FIG. 12(a) and (b), even when pressure is applied to the load position P1 of the flexure element (FIG. 12(a)) or to the load position P2 (FIG. 12(b)), the strain magnitude is highest in a region around the load portion, and the strain magnitude decreases concentrically outward from the region, forming this distribution. Since a peripheral portion of the flexure element (in the figure, a portion at a radius of 6.5 mm or more from the center) is fixed to the housing, the strain magnitude is especially low, and the strain magnitude increases slightly in the vicinity of the fixed portion. However, except for the fixed portion and the vicinity of the portion, the distribution of strain magnitudes that generally decreases concentrically from the center remains unchanged.

As described above, by providing the cover member in the pulse wave sensor, even if the pulse wave sensor (the cover member of the pulse wave sensor) is not applied to a measurement target at an appropriate position and angle, the strain magnitude can be concentrated on the load portion (or the center of the base) in the same manner as when the pulse wave sensor (the cover member of the pulse wave sensor) is applied at the appropriate position and angle. Thus, by providing the cover member in the pulse wave sensor, it can be said that a biological signal detection range is expanded.

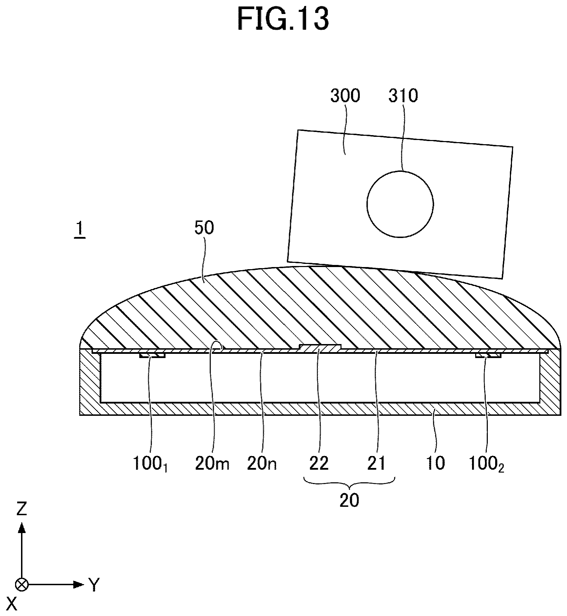

In this arrangement, when the pulse wave sensor is applied to a body (for example, wrist) of the measurement target, for example, even if the pulse wave sensor is applied to the skin diagonally at a slight angle as shown in FIG. 13 rather than vertically as shown in FIG. 2, pressure from the artery 310 can be detected with accuracy that allows the pulse wave to be detected. In other words, the pulse wave sensor that is equipped or formed with the cover member can expand the measurable range. In other words, the pulse wave sensor equipped or formed with the cover member can smooth the difference in detection sensitivity, depending on the position of flexure element portions of the pulse wave sensor.

Moreover, when the pulse wave sensor is provided with the cover member, the pulse wave can be properly measured even if an angle and position of the pulse wave sensor that is applied to a body are not accurate. As a result, the effect of making measurement easier for a user of the pulse wave sensor is obtained. Further, since a position where the pulse wave sensor is applied is less to be finely adjusted, the pulse wave can be measured more quickly using the pulse wave sensor. Therefore, the effect of the overall measurement time being reduced can be obtained. In addition, reproducibility and stability of pulse wave measurement are improved.

### [Strain Gauge 100]

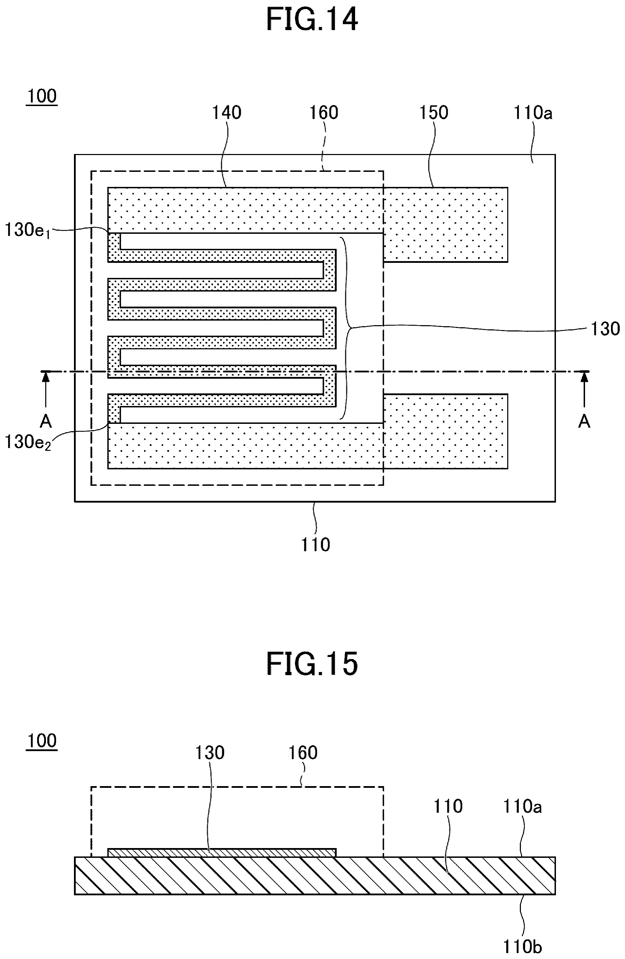

Hereinafter, a strain gauge 100 will be described. FIG. 14 is a plan view illustrating the strain gauge according to the first embodiment. FIG. 15 is a cross-sectional view (part 1) illustrating the strain gauge according to the first embodiment, illustrating a cross section along the line A-A in FIG. 14.

Referring to FIGS. 14 and 15, the strain gauge 100 includes a substrate 110, a resistor 130, lines 140, electrodes 150, and a cover layer 160. The cover layer 160 can be provided as needed. In FIGS. 14 and 15, only an outer edge of the cover layer 160 is shown by a dashed line for convenience. First, each component of the strain gauge 100 will be described in detail as follows.

In the description of the strain gauge using FIGS. 14 to 16, definitions of the upper surface and lower surface are different from those in other figures. Specifically, in FIGS. 14 to 16, for convenience, a side of the strain gauge 100 where the resistor 130 of the substrate 110 is provided is referred to as an "upper side," and a side of the strain gauge 100 where the resistor 130 is not provided is referred to as a "lower side." In addition, a surface located on the upper side of each component is referred to as the "upper side," and a surface located on the lower side of each component is referred to as the "lower side." However, the strain gauge 100 can be used in an inverted state. In addition, the strain gauge 100 can be arranged at any angle. A plan view in the description of FIGS. 14 to 16 means that a target is viewed in a direction normal to an upper surface 110a of the substrate 110 from the upper side to the lower side. A planar shape in the description of FIGS. 14 to 16 refers to a shape of the object when the object is viewed in the normal direction. The strain gauge 100 is attached to a second surface 20n of the flexure element 20 such that the substrate 110 faces the second surface 20n of the flexure element 20.

The substrate 110 is a member that serves as a base layer for forming the resistor 130 and the like. The substrate 110 has flexibility. The thickness of the substrate 110 is not particularly limited, and may be appropriately determined according to the purpose or the like of use of the strain gauge 100. For example, the thickness of the substrate 110 may be about 5 µm to 500 µm. The thickness of the substrate 110 is preferably within the range of 5 µm to 200 µm from the viewpoint of the transmissibility of strain from the second surface 20n of the flexure element 20 to a receiving portion and of dimensional stability against environmental changes. The thickness of the substrate 110 is preferably 10 µm or more from the viewpoint of the insulating property.

The substrate 110 is formed of an insulating resin film such as PI (polyimide) resin, epoxy resin, PEEK (polyetheretherketone) resin, PEN (polyethylene naphthalate) resin, PET (polyethylene terephthalate) resin, PPS (polyphenylene sulfide) resin, LCP (liquid crystal polymer) resin, polyolefin resin, and the like. The film refers to a flexible member having a thickness of about 500 µm or less.

When the substrate 110 is formed of an insulating resin film, the insulating resin film may contain fillers, impurities, and the like. For example, the substrate 110 may be formed of an insulating resin film containing fillers such as silica or alumina.

Materials other than resins for the substrate 110 include, for example, crystalline materials such as SiO₂, ZrO₂ (including YSZ), Si, Si₂N₃, Al₂O₃ (including sapphire), ZnO, and perovskite ceramics (CaTiO₃ or BaTiO₃). In addition to the above crystalline materials, amorphous glass or the like may be used as a material for the substrate 110. Metals such as aluminum, aluminum alloy (duralumin), and titanium may be used as a material for the substrate 110. When a metallic substrate 110 is used, an insulating film is provided to cover the upper surface 110a.

The resistor 130 is a thin film formed in a predetermined pattern on the upper side of the substrate 110. In the strain gauge 100, the resistor 130 is a sensitive portion that generates a resistance change in response to receiving strain. The resistor 130 may be formed directly on the upper surface 110a of the substrate 110 or on the upper surface 110a of the substrate 110 via another layer. In FIG. 14, for convenience, the resistor 130 is shown in a stippled pattern.

The resistor 130 has a structure in which a plurality of elongated portions are arranged at predetermined intervals in their longitudinal directions aligned in the same direction (the direction of the line A-A in the example of FIG. 14), in which end portions of adjacent elongated portions are mutually connected, and in which the plurality of elongated portions are folded back in a zigzag manner as a whole. The longitudinal direction of the plurality of elongated portions is a grid direction, and a direction perpendicular to the grid direction becomes a grid width direction (the direction perpendicular to the A-A line in the example of FIG. 14).

The longitudinal ends of two elongated portions located outermost in the grid width direction bend in the grid width direction to form respective ends 130e₁ and 130e₂ in the grid width direction of the resistor 130. Each of the ends 130e₁ and 130e₂ in the grid width direction of the resistor 130 is electrically connected to the electrode 150 via the line 140. In other words, the line 140 electrically connects each of the ends 130e₁ and 130e₂ in the grid width direction of the resistor 130 with each electrode 150.

The resistor 130 can be formed of, for example, a material including Cr (chromium), a material containing Ni (nickel), or a material containing both Cr and Ni. That is, the resistor 130 can be formed of a material containing at least one of Cr or Ni. The material containing Cr includes, for example, a Cr mixed-phase film. As a material containing Ni, for example, Cu-Ni (copper nickel) can be used. The material containing both Cr and Ni includes, for example, Ni-Cr (nickel chromium).

Here, the Cr mixed-phase film is a film in which Cr, CrN, and Cr₂N, and the like are mixed. The Cr mixed-phase film may contain unavoidable impurities such as chromium oxide.

The thickness of the resistor 130 is not particularly limited and may be appropriately determined in accordance with the purpose or the like of use of the strain gauge 100. For example, the thickness of the resistor 130 may be about 0.05 µm to 2 µm. In particular, when the thickness of the resistor 130 is 0.1 µm or more, crystallinity (for example, crystallinity of α-Cr) of the crystals that constitute the resistor 130 is improved. When the thickness of the resistor 130 is 1 µm or less, the occurrence of (i) cracks in the film, and (ii) warpage from the substrate 110 of the film, caused by the internal stress of the film constituting the resistor 130 are reduced.

In view of preventing lateral sensitivity and preventing disconnection, the width of the resistor 130 is preferably greater than or equal to 10 µm and less than or equal to 100 µm. Furthermore, the width of the resistor 130 is preferably greater than or equal to 10 µm and less than or equal to 70 µm, and more preferably greater than or equal to 10 µm and less than or equal to 50 µm.

For example, when the resistor 130 is a Cr mixed-phase film, the stability of gauge characteristics can be improved by using α-Cr (alpha chromium), which is a stable crystalline phase, as a main component. For example, when the resistor 130 is a Cr mixed-phase film, the gauge rate of the strain gauge 100 can be 10 or more, and the temperature coefficient of strain gauge rate TCS and the temperature coefficient of resistance TCR can be set within the range of -1,000 ppm/°C to +1,000 ppm/°C by using α-Cr as the main component in the resistor 130. Here, the "main component" means a component that accounts for 50 wt% or more of all materials constituting the resistor. From the viewpoint of improving the gauge characteristics, it is preferable that the resistor 130 contains 80 wt% or more of α-Cr. Further, from the same viewpoint, it is more preferable that the resistor 130 contains 90 wt% or more of α-Cr. Note that α-Cr is Cr having a bcc structure (body-centered cubic lattice structure).

When the resistor 130 is a Cr mixed-phase film, CrN and Cr₂N contained in the Cr mixed-phase film are preferably at 20 wt% or less. When CrN and Cr₂N contained in the Cr mixed-phase film are at 20 wt% or less, it is possible to suppress decreases in the gauge rate of the strain gauge 100.

The ratio of CrN to Cr₂N in the Cr mixed-phase film is preferably set so that the proportion of Cr₂N is greater than or equal to 80 wt% and less than 90 wt% relative to the total weight of CrN and Cr₂N. More preferably, the ratio is set so that the proportion of Cr₂N is 90 wt% or more and less than 95 wt% relative to the total weight of CrN and Cr₂N. Here, Cr₂N has semiconducting properties. Therefore, by setting the ratio of Cr₂N to 90 wt% or more and less than 95 wt%, reductions in TCR (negative TCR) becomes more remarkable. Furthermore, by setting the ratio of Cr₂N to greater than or equal to 90 wt% and less than 95 wt%, ceramization of the resistor 130 can be reduced, and brittle fracture of the resistor 130 hardly occurs.

On the other hand, CrN has the advantage of being chemically stable. By including more CrN in the Cr mixed-phase film, the possibility of unstable N generation can be reduced, and as a result, a stable strain gauge can be obtained. Here, the "unstable N" means a trace amount of N₂ or atomic N that may exist in the Cr mixed-phase film. These unstable N may escape from the film, depending on the external environment (e.g., high temperature environment). The film stress of the Cr mixed-phase film may change when the unstable N escapes from the film.

In the strain gauge 100, when the Cr mixed-phase film is used as the material of the resistor 130, high sensitivity and size reduction can be realized. For example, while the output of a conventional strain gauge is about 0.04 mV/2V, an output of 0.3 mV/2V or more can be obtained when the Cr mixed-phase film is used as the material of the resistor 130. In addition, while the size (gauge length × gauge width) of the conventional strain gauge is about 3 mm × 3 mm, the size (gauge length × gauge width) of the Cr mixed-phase film can be reduced to about 0.3 mm × 0.3 mm when the Cr mixed-phase film is used as the material of the resistor 130.

The lines 140 are provided on the substrate 110. Each line 140 is electrically connected to the resistor 130 and the electrode 150. The line 140 is not limited to a linear shape and can have any pattern. The line 140 can have any width and any length. In FIG. 14, for convenience, the line 140 is illustrated with a stippled pattern having density lower than the resistor 130.

The electrodes 150 are provided on the substrate 110. The electrodes 150 are electrically connected to the resistor 130 via the lines 140. In plan view, the electrodes 150 are each wider than the line 140 to have a substantially rectangular shape. The electrodes 150 are a pair of electrodes that externally output the change in the resistance value of the resistor 130 according to strain. For example, a lead wire or the like for an external connection is joined to the electrode 150. A metal layer having a low resistance such as copper, or a metal layer having good solderability such as gold may be laminated on the upper surface of the electrode 150. Although the resistor 130, the line 140, and the electrode 150 are indicated by different symbols for convenience, these can be formed integrally by the same material in the same process. In FIG. 14, for convenience, the electrodes 150 are illustrated with a stippled pattern having the same density as the lines 140.

The cover layer 160 (protective layer) is provided on and above the upper surface 110a of the substrate 110, as necessary, so as to cover the resistor 130 and the lines 140 and expose the electrodes 150. Examples of materials of the cover layer 160 include insulating resins such as PI resin, epoxy resin, PEEK resin, PEN resin, PET resin, PPS resin, and composite resin (for example, silicone resin, polyolefin resin). The cover layer 160 may contain fillers or pigments. The thickness of the cover layer 160 is not particularly limited and can be appropriately selected according to the purpose. For example, the thickness of the cover layer 160 can be about 2 µm to 30 µm. By providing the cover layer 160, the occurrence of mechanical damage or the like to the resistor 130 can be suppressed. Moreover, by providing the cover layer 160, the resistor 130 can be protected from moisture or the like.

### [Method of Manufacturing Strain Gauge 100₁

In the strain gauge 100 according to the present embodiment, the resistor 130, the lines 140, the electrodes 150, and the cover layer 160 are formed on or above the substrate 110. Another layer (such as a functional layer described later) may be formed between the substrate 110 and these component layers.

The method of manufacturing the strain gauge 100 will be described below. In order to manufacture the strain gauge 100, first, the substrate 110 is prepared, and a metal layer (for convenience, referred to as a metal layer A) is formed on the upper surface 110a of the substrate 110. The metal layer A is a layer that is ultimately patterned to become the resistor 130, the lines 140, and the electrodes 150. In this arrangement, the material and thickness of the metal layer A are the same as those of the resistor 130, the line 140, and the electrode 150.

The metal layer A can be formed by, for example, magnetron sputtering by using a raw material target capable of forming the metal layer A. Instead of the magnetron sputtering, the metal layer A may be formed by reactive sputtering, vapor deposition, arc ion plating, or pulsed laser deposition. After the metal layer A is deposited on the upper surface 110a of the substrate 110, the metal layer A is patterned by well-known photolithography into a planar shape similar to that of the resistor 130, lines 140, and electrodes 150 in FIG. 14.

The metal layer A may be formed after an underlayer is formed on the upper surface 110a of the substrate 110. For example, a functional layer having a predetermined film thickness may be vacuum-deposited on the upper surface 110a of the substrate 110 by conventional sputtering. By providing the underlayer with such an approach, the gauge characteristics of the strain gauge 100 can be stabilized.

In the present application, the functional layer refers to a layer having a function of promoting crystal growth of the metal layer A (resistor 130) that is used as at least an upper layer. It is preferable that the functional layer further has a function of preventing oxidation of the metal layer A due to oxygen or moisture contained in the substrate 110, and/or improving adhesion between the substrate 110 and the metal layer A. The functional layer may further have other functions.

An insulating resin film constituting the substrate 110 may contain oxygen and moisture, and Cr may form a self-oxidation film. In this case, especially when the metal layer A contains Cr, it is preferable to form the functional layer having a function of preventing oxidation of the metal layer A.

In this arrangement, by providing the functional layer under the metal layer A, crystal growth of the metal layer A can be promoted, and the metal layer A that has a stable crystalline phase can be produced. As a result, the stability of the gauge characteristics in the strain gauge 100 is improved. Moreover, the material that constitutes the functional layer diffuses into the metal layer A, and the gauge characteristic in the strain gauge 100 is improved.

Examples of the material of the functional layer include one or more metals selected from the group consisting of Cr (chromium), Ti (titanium), V (vanadium), Nb (niobium), Ta (tantalum), Ni (nickel), Y (yttrium), Zr (zirconium), Hf (hafnium), Si (silicon), C (carbon), Zn (zinc), Cu (copper), Bi (bismuth), Fe (iron), Mo (molybdenum), W (tungsten), Ru (ruthenium), Rh (rhodium), Re (rhenium), Os (osmium), Ir (iridium), Pt (platinum), Pd (palladium), Ag (silver), Au (gold), Co (cobalt), Mn (manganese), and Al (aluminum); alloys of any of the metals in this group; or compounds of any of the metals in this group.

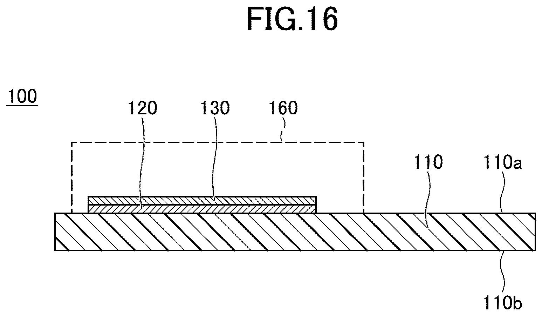

FIG. 16 is a cross-sectional view (part 2) illustrating the strain gauge according to the first embodiment. FIG. 16 shows the cross-sectional shape of the strain gauge 100 when the functional layer 120 is provided as the underlayer of the resistor 130, the lines 140, and the electrodes 150.

The planar shape of the functional layer 120 may be patterned substantially the same as, for example, the planar shape of the resistor 130, the lines 140, and the electrodes 150. However, the planar shape of the functional layer 120, and the planar shape of the resistor 130, the lines 140, and the electrodes 150 need not be substantially the same. For example, when the functional layer 120 is formed of an insulating material, the functional layer 120 may be patterned differently from the planar shape of the resistor 130, the lines 140, and the electrodes 150. In this case, the functional layer 120 may be formed in a solid state in a region where, for example, the resistor 130, the lines 140, and the electrodes 150 are formed. Alternatively, the functional layer 120 may be formed as a solid layer over the entire upper surface of the substrate 110.

After the resistor 130, the lines 140, and the electrodes 150 are formed, the cover layer 160 is formed on and above the upper surface 110a of the substrate 110 as necessary. The cover layer 160 covers the resistor 130 and the lines 140, but the electrodes 150 may be exposed from the cover layer 160. For example, the cover layer 160 can be formed by laminating a semicured thermosetting insulating resin film on the upper surface 110a of the substrate 110, so as to cover the resistor 130 and the lines 140 and expose the electrodes 150, and then by heating and curing the insulating resin film. With the above process, the strain gauge 100 is completed.

In the above description, an example of using a strain gauge has been provided as an example of a detection element, but a piezoelectric element can also be used as the detection element. The types of piezoelectric elements are not particularly limited. When the piezoelectric element is used as the detection element, an arrangement position of the piezoelectric element in the flexure element may be appropriately determined according to the direction of a force detected by the piezoelectric element, and detection accuracy. Further, the configuration other than the piezoelectric element (for example, a flexure element and a cover member) may be the same as that in a case of using a strain gauge, or may be appropriately changed according to the type of piezoelectric element.

Although the preferred embodiments and the like have been described in detail above, the above embodiments and the like are not limited. Various modifications and substitutions can be made to the embodiments and the like without departing from the scope of claims.

This international application claims priority to U.S. Patent Application No. 63/493,109, filed on March 30, 2023, and Japanese Patent Application No. 2023-124909, filed on July 31, 2023, the entire contents of which are incorporated herein by reference.

### REFERENCE SIGNS LIST

1, 1A, 1B, 1C, 1D pulse wave sensor, 10 housing, 20 flexure element, 20m first surface, 20n second surface, 21 base, 22 load portion, 50, 50A, 50B, 50C, 50D cover member, 51 recess, 52 protrusion, 90 gap, 100₁, 100₂, 100₃, 100₄ strain gauge, 110 substrate, 110a top surface, 130 resistor, 140 line, 150 electrode, 160 cover layer, 130e₁, 130e₂ end portion

## Claims

1. A pulse wave sensor to be used in contact with a body of a measurement target, comprising:
a flexure element having a first surface that is on a measurement target side, and a second surface located opposite to the first surface;
at least one detection element disposed on the second surface, and configured to detect strain of the flexure element and/or pressure applied to the flexure element; and
a cover member mounted or formed on the first surface, the cover member including:
a recess opening toward the first surface of the flexure element, and
a protrusion having a surface that faces the first surface of the flexure element, the protrusion protruding toward the first surface of the flexure element beyond the recess,
wherein a gap is formed between the recess and the first surface of the flexure element, and
wherein the pulse wave sensor is configured to detect a pulse wave based on a detection value of the detection element.

2. The pulse wave sensor according to claim 1, wherein a surface of the cover member on the measurement target side is a curved surface that is highest at a central portion of the first surface of the flexure element and gradually decreases toward a peripheral portion of the first surface of the flexure element, with reference to the first surface of the flexure element.

3. The pulse wave sensor according to claim 1 or 2, wherein the protrusion and the first surface of the flexure element are bonded to each other.

4. The pulse wave sensor according to claim 1 or 2, wherein the protrusion and the first surface of the flexure element are separated from each other, and are arranged to contact each other, upon occurrence of a condition in which the pulse wave sensor contacts the body of the measurement target.

5. The pulse wave sensor according to claim 1 or 2, wherein the flexure element includes a load portion protruding from a central portion of the first surface toward the measurement target side, and
wherein the protrusion is disposed at a position that overlaps with the load portion in plan view.

6. The pulse wave sensor according to claim 5, wherein the protrusion and the load portion are bonded to each other.

7. The pulse wave sensor according to claim 5, wherein the protrusion and the load portion are separated from each other, and are arranged to contact with each other, upon occurrence of a condition in which the pulse wave sensor contacts the body of the measurement target.

8. The pulse wave sensor according to any one of claims 1 to 7, wherein the cover member has a curved surface curving toward the first surface, the curved surface serving as the protrusion at at least a portion of the surface of the cover member facing the first surface of the flexure element.

9. The pulse wave sensor according to any one of claims 1 to 8, wherein the flexure element is made of glass.

10. The pulse wave sensor according to claim 9, wherein fracture strength of the flexure element is greater than 20 N under static pressure.

11. The pulse wave sensor according to claim 9 or 10, wherein the flexure element is made of chemically reinforced glass.

12. The pulse wave sensor according to any one of claims 1 to 11, wherein the at least one detection element includes a plurality of strain gauges, and
wherein the pulse wave sensor is configured to detect the pulse wave based on a change in resistance values of the plurality of strain elements.

13. The pulse wave sensor according to claim 12, wherein the plurality of strain gauges include:
a pair of strain gauges configured to detect compressive strain of the flexure element, and
another pair of strain gauges configured to detect tensile strain of the flexure element,
wherein the pair of strain gauges and the other pair of strain gauges are coupled to constitute sides of a bridge circuit, and
wherein a signal indicating the pulse wave is to be generated by the bridge circuit.
